# EUROPEAN PATENT SPECIFICATION

(11) **EP 4 291 629 B1**
(45) Date of publication and mention of the grant of the patent: **18.12.2024**
(21) Application number: 22710432.0
(22) Date of filing: 10.02.2022
(51) Int. Cl.: C12M 1/00, A23F 3/10, C12C 11/00

(54) **METHOD AND DEVICE FOR THE INDUSTRIAL PRODUCTION OF KOMBUCHA**
VERFAHREN UND VORRICHTUNG ZUR INDUSTRIELLEN HERSTELLUNG VON KOMBUCHA
PROCÉDÉ ET DISPOSITIF DE PRODUCTION INDUSTRIELLE DE KOMBUCHA

(30) Priority: 11.02.2021 BE 202105100
(43) Date of publication of application: 20.12.2023
(73) Proprietor: Duvel Moortgat NV, 2870 Puurs-Sint-Armands (BE)
(72) Inventor: MOORTGAT, Michel, 2870 Puurs-Sint-Amands (BE); BRITTON, Scott, 2870 Puurs-Sint-Amands (BE); NEVEN, Hedwig, 2870 Puurs-Sint-Amands (BE)
(74) Representative: Brantsandpatents bv
(86) International application number: PCT/IB2022/051192
(87) International publication number: WO 2022/172185

(56) References cited:
- EP-A2- 0 089 225
- CN-A- 108 753 592
- CN-A- 110 892 928
- US-A1- 2003 097 937
- US-A1- 2004 149 137

## Description

### TECHNICAL FIELD

The invention relates to a method for the industrial production of a beverage based on a fermented liquid such as tea.

In a second aspect, the invention also relates to a device for the industrial production of a beverage based on fermented liquid.

The invention can be applied, among other things, in the production of kombucha.

### PRIOR ART

Methods and devices for the industrial production of a beverage based on fermented liquids such as tea are known per se from the prior art. The production of a drink based on fermented tea is done by dissolving sugar in boiling water, wherein tea leaves are infused with the boiling sugar mixture. After cooling to room temperature, a mixture of symbiotic bacteria and yeasts is added, after which the fermentation sta rts.

During the production of kombucha or other fermented drinks, a floating layer forms on the liquid, in the form of a gelatinous cellulose-based biofilm. This gelatinous cellulose-based biofilm is called "SCOBY" (Symbiotic Culture of Bacteria and Yeast), and it is generally required for the fermentation process. However, the presence of the biofilm makes it extremely difficult to produce fermented liquid on a large scale. After all, this biofilm must be removed from the mixture after fermentation, where removal often has to be done manually. This is very time consuming, requires intensive manual labor and the presence of biofilm causes additional problems, such as clogging of input or output of production vessels.

The production of fermented liquid is generally an aerobic process, whereby a standard device for the production of fermented liquid comprises an open container with a still liquid. However, this leads to problems in sufficiently removing a biofilm, causing contamination of the container, and a limited production capacity. A contamination can be physical, chemical or biological in nature. In addition, there is also a need for better ways to remove particles from the liquid after production. Insufficiently clarified liquid (tea in which too many particles or microorganisms remain) has negative effects on the shelf life, taste and the build-up of alcohol and/or CO2 after bottling.

EP1718727 describes a device wherein pure oxygen is injected into a closed fermentation vessel during the fermentation. In this case, the fermentation vessel is homogenized mechanically by means of an agitator. External oxygen is added to the fermentation vessel. The device does not disclose use for fermenting tea.

CN207294776 describes a device wherein a closed fermentation vessel is equipped with a stirring mechanism. According to the device, the stirring mechanism is connected to an air pump to introduce filtered air into the fermentation vessel. The device does not disclose use for fermenting tea.

An adapted method for the production of fermented tea is known, for example, from CN103416545. The method describes a production of kombucha in a tank wherein a woven fabric is used to capture and remove the formed cellulose-based biofilm during the fermentation process. The woven fabric is moved by means of rollers through the interior of the fermentation tank, from a starting point at the top of the tank to an end point at the bottom of the tank.

Also known is the method relating to an industrial production of kombucha from US20200270556 and WO2018204243.

US20200270556 describes a method to produce kombucha on a large scale, using a fermentation system based on vertically oriented fermentation stacks. The fermentation stacks allow oxygen supply from the outside air and are heat conducting.

WO2018204243 describes a method of producing an alcoholic beverage based on kombucha, using a fermentation system based on the use of separate fermentation tanks for the production of alcoholic tea liqueur on the one hand and the production of fermented tea on the other hand. Both productions are then combined to obtain an alcoholic kombucha drink with a specific alcohol percentage, CN 110892928 A describes a method to produce kombucha using fermentation of a sugar-containing liquid, wherein the fermented liquid is transported between different reactors corresponding to different fermentation steps, wherein each fermentation reactor is provided with nozzles allowing oxygen injection in the fermentation broth.

A difficulty with the above known devices and methods is that the cellulose-based biofilm (SCOBY) is still formed. This makes these known methods less suitable for industrially producing kombucha. After all, the biofilm formed by bacteria, yeast and cellulose in the fermentation broth is difficult to remove from fermentation vessels once formed, due to its size, weight and complex structure. Often this biofilm has to be manually removed from the fermentation vessels periodically. If not removed, this biofilm can significantly reduce tank efficiency by preventing proper cleaning of fermentation vessels, significantly reducing the efficiency of downstream separation through the centrifuge and preventing product sterilization through membrane filtration.

The present invention aims to solve at least some of the above problems or drawbacks.

### SUMMARY OF THE INVENTION

In a first aspect, the present invention relates to a method for the industrial production of a beverage based on fermented liquid according to claim 1. The method comprises fermenting liquid in a closed fermentation tank, by means of microorganisms. In this case, the fermenting liquid is circulated in the fermentation tank, wherein at least a portion of the fermenting liquid is mixed with oxygenated air outside the fermentation tank, and then reintroduced into the fermentation tank.

This is particularly advantageous because the combination of circulation and aeration of the fermenting liquid prevents the formation of a cellulose-based biofilm in the tank. This eliminates the need to remove the SCOBY at the end of the fermentation process, which of course improves the efficiency of the process. Usually, the removal of such a biofilm is a complex and labor-intensive process, as it has to be removed manually. Moreover, in the present invention, it becomes possible to thoroughly clean the fermentation tank, which is advantageous for aseptic production reducing contamination and food safety issues.

Production efficiency is also increased when no more biofilm is formed during fermentation. The presence of biofilm often clogs the input and output conduits in the fermentation tank, preventing the fermentation tank from being emptied without periodically removing the biofilm. A worker will usually manually ensure that no biofilm can clog the conduits, but these actions slow down the production process.

Preferred forms of the method are set out in claims 2-12.

According to a preferred form, a synergistic effect takes place through the combination of a circulation and an oxygenation. The circulation in the fermentation tank takes place here by means of a jet head, preferably a rotary jet head. In this way, after mixing with oxygenated air outside the tank, the fermentation of liquid is carried out more efficiently throughout the fermentation tank.

In a second aspect, the present invention relates to a device for the industrial production of fermented liquid according to claim 13.

The device comprises a closed fermentation tank internally provided with a jet head. The fermentation tank is herein provided with a first conduit which is suitable for transporting at least a part of the fermenting drink from the fermentation tank to an aerator or oxygenator located outside the fermentation tank. The first conduit opens into the aerator or oxygenator and a second conduit exits from the aerator or oxygenator and opens into an inlet of the jet head located inside the fermentation tank, wherein the first and second conduits form an external loop located outside the fermentation tank and wherein the jet head is provided with perforations.

The circulation in the fermentation tank is done by means of a jet head. This can be a stationary jet head. In a preferred form it is a rotary jet head. The presence of the jet head helps to prevent the formation of a cellulose-based biofilm in the fermentation tank. Moreover, the formation of a cellulose-based biofilm causes blockages in the conduits connected to the tank, which prevents the tank from being optimally emptied and cleaned. The circulation in the tank eliminates these problems. An additional advantage of using a jet head is that the fermenting liquid, after mixing with oxygenated air in an oxygenator outside the tank, is more efficiently distributed throughout the fermentation tank.

Preferred forms of the device are described in dependent claims 14 and 15.

According to a preferred form, the device comprises a centrifuge, connected to the fermentation tank, for centrifuging a fermented liquid. This allows particles with a certain density or weight (such as small biofilm residues) to be removed from the liquid. This better clarifies and prepares the fermented liquid for further filtration.

According to a preferred form, the device comprises a filter unit for filtering the fermented liquid. This is particularly advantageous because the filter unit removes microorganisms such as bacteria and yeasts from the liquid. This is beneficial for shelf life and food safety.

### BRIEF DESCRIPTION OF THE DRAWINGS

**Figure 1** shows a schematic representation of a device for the production of fermented tea according to the present invention.
**Figure 2** shows a schematic representation of an embodiment of the jet head according to the present invention.

### DETAILED DESCRIPTION

Unless otherwise defined, all terms used in the description of the invention, including technical and scientific terms, have the meaning as commonly understood by a person skilled in the art to which the invention pertains. For a better understanding of the description of the invention, the following terms are explained explicitly.

In this document, "a" and "the" refer to both the singular and the plural, unless the context presupposes otherwise. For example, "a segment" means one or more segments.

When the term "around" or "about" is used in this document with a measurable quantity, a parameter, a duration or moment, and the like, then variations are meant of approx. 20% or less, preferably approx. 10% or less, more preferably approx. 5% or less, even more preferably approx. 1% or less, and even more preferably approx. 0.1% or less than and of the quoted value, insofar as such variations are applicable in the described invention. However, it must be understood that the value of a quantity used where the term "about" or "around" is used, is itself specifically disclosed.

The terms "comprise", "comprising", "consist of", "consisting of", "provided with", "include", "including", "contain", "containing", are synonyms and are inclusive or open terms that indicate the presence of what follows, and which do not exclude or prevent the presence of other components, characteristics, elements, members, steps, as known from or disclosed in the prior art.

Quoting numerical intervals by endpoints comprises all integers, fractions and/or real numbers between the endpoints, these endpoints included.

In a first aspect, the present invention relates to a method for the industrial production of a beverage based on fermented liquid. The method comprises fermenting a liquid such as a tea or wort in a closed fermentation tank, by means of microorganisms. Wort is the clear sugary, aqueous solution obtained after mashing, the brewing process wherein the starch of malt and any other grains is enzymatically broken down into simpler molecules. In this case, the fermenting liquid is circulated in the fermentation tank, wherein at least a portion of the fermenting liquid is mixed with oxygen or oxygenated air outside the fermentation tank, and then reintroduced into the fermentation tank.

The combination of circulation of the fermenting liquid with mixing of oxygenated air (oxygenation) prevents the formation of a cellulose-based biofilm in the tank. The fermentation of liquid such as tea or wort preferably takes place by means of a symbiotic culture of bacteria and yeast. The symbiotic culture, according to the prior art, results in the formation of a cellulose-based biofilm (SCOBY) which floats on the liquid. According to the present invention, the formation of a biofilm is prevented. In addition, it becomes possible to thoroughly clean a fermentation tank, which is advantageous for aseptic production reducing contamination and food safety issues. An additional advantage of the combination of circulation of the fermenting liquid with mixing of oxygenated air is that the acidification process, the process by which alcohol is converted into an acid (e.g., lactic acid or acetic acid), is more efficient, which has a positive influence on the composition and taste of the fermented liquid.

In addition, the production efficiency is increased when no more biofilm is formed during fermentation. The presence of biofilm often clogs the input and output conduits in a fermentation tank, preventing the fermentation tank from being emptied without periodically removing the biofilm. Biofilm removal is usually done manually, requiring significant time and labor. The present invention eliminates this problem.

Mixing the fermenting liquid with oxygen or oxygenated air is also advantageous because it allows an aerobic fermentation process to be carried out within a closed tank. By carrying out the mixing outside the fermentation tank, the concentration of oxygen that is added can be strictly controlled, in order to optimize the fermentation in this way.

According to the invention, circulation in the fermentation tank is accomplished by means of a jet head. A jet head is connected to a conduit provided with an inlet valve through which the liquid is mixed with air and provided with perforations through which the fermenting liquid is passed.

In a preferred form, use is made of a Venturi wherein the inner wall of the jet head comprises a constriction which generates a fluid pressure in the jet head. This liquid pressure is then transferred through the perforations in the jet head to the liquid in the fermentation tank, creating a circulation. The use of such a jet head is advantageous because the oxygenated fermenting liquid is optimally circulated.

According to the invention, the mixing of the liquid infusion with air (the oxygenation) takes place outside the fermentation tank, with a portion of the fermenting liquid being conveyed by means of conduits provided for that purpose, which are in liquid connection with the fermentation tank, to an inlet valve (an aerator or oxygenator), where oxygenated air is mixed with the fermenting liquid, and then conveyed back to the fermentation tank via a conduit. In an embodiment, a connection is provided to a tank with oxygenated air).

According to an embodiment, the oxygen content in the aerated or oxygenated liquid is between 50 and 8000 ppb. Modulation of the oxygen content is ideally suited to control the fermentation process, whereby an adjustment of the oxygen content can cause more acidification (formation of acids) on the one hand or can stimulate the formation of a larger biomass on the other hand.

According to a further embodiment, the conduit which introduces the aerated or oxygenated liquid back into the fermentation tank discharges into an inlet of the jet head. The aerated or oxygenated liquid is hereby circulated in the fermentation tank by means of the jet head.

According to an embodiment, fermented liquid is subjected to one or more separation processes after fermentation, wherein particles of a certain size or density are removed from the fermented liquid. An attempt is made to remove microbial residues that are still present in the fermented liquid, in order to prevent an unwanted additional fermentation after production.

In an embodiment, the fermented drink is bottled for use after the fermentation process. This so-called "raw" drink such as "raw" kombucha will not undergo any further treatment steps after fermentation, other than those aimed at transport, bottling and consumption.

According to another embodiment, the fermented liquid undergoes at least two separation steps, wherein a first separation is done by centrifugation and a second separation is done by a filter unit. This removes any impurities that could affect the final quality of the product. The present invention uses two separation steps to clarify the fermented liquid: a centrifugation step and a filtration step. A centrifuge provides a density separation in which solid components are removed from a liquid by means of a centrifugal force. A filter unit uses a physical separation by means of filters of defined diameter to remove microorganisms and contaminants, in order to achieve a certain sterilization.

According to a further embodiment, the fermented liquid undergoes a second separation by means of a filter unit, wherein the filter unit comprises at least three filters. Preferably, a pore size of the filters in the filter unit is between 0.2 and 20 microns.

In a further preferred form, a first filter will be suitable for filtering larger particles present in the fermented liquid, such as for instance aggregates of biofilm. Preferably, this first filter will have a pore size between 5 and 10 microns. In a further preferred form, a second filter will have a pore size between 1 and 5 microns and a 0.40-0.48-micron filter is used as third filter to remove all yeasts and larger bacteria, where a 0.45-micron filter is capable of removing 10^{e}5 CFU/cm² from a liquid as standard). More preferably, a 0.20-0.28-micron filter is used to remove all bacteria and achieve better sterility.

In another embodiment, as an alternative to filtration and after centrifugation, the fermented beverage may undergo a pasteurization step. In a preferred form this will be a flash pasteurization or HTST pasteurization. In HTST pasteurization (High Temperature/Short Time) a liquid is brought to a certain temperature (for example 72°C) for a certain period (for example at least 15 seconds).

According to an embodiment, the fermented drink is a tea infusion prepared from green, oolong or black tea to which sugar, a sugar solution or other sacchariferous, natural sweetener (such as, for example, honey, agave, date syrup, maple syrup) has been added. Preferably, the sugar is a monosaccharide (such as glucose, fructose or galactose) or a disaccharide (such as sucrose, lactose or maltose) or a mixture thereof.

In another embodiment, the fermented drink is wort or other sacchariferous liquid such as a fruit juice.

A starter culture of microorganisms is then added to conduct the fermentation process. A starter culture is preferably a symbiotic aerobic culture of bacteria and yeasts, wherein the bacteria are, for example, Gram-negative AAB bacteria (such as Acetobacter, Gluconobacter or Komagataeibacter) or Gram-positive LAB bacteria (such as Lactobacillus), and wherein yeasts are, for example, Saccharomyces or Zygosaccharomyces. It goes without saying that other microorganisms known in the prior art are also options.

Yeasts transform the sugars in the infusion into alcohol and CO2, and then the bacteria convert the formed alcohol into acids (such as lactic acid or acetic acid). The present invention is advantageous because the acidification process (the forming of acids) is enhanced in large fermentation tanks, by the method by which the aerobic culture is performed. After all, by mixing the symbiotic culture of bacteria and yeasts with oxygen and by circulating the fermenting liquid in the tank by means of the jet head, the conversion process of sugar to acid is optimized, so that the fermented liquid obtains a better acidity. This allows production of fermented liquid such as tea on an industrial scale.

According to an embodiment, the fermentation of a liquid proceeds by means of an aerobic process.

According to an embodiment, the fermentation of liquid takes place at a temperature between 18 and 28°C, wherein the fermentation time is preferably 8 to 14 days. Preferably, the fermentation takes place at a temperature between 20 and 25°C for 8 to 12 days. More preferably, the fermentation takes place at a temperature of 23°C for 9 to 10 days. The temperature at which the fermentation takes place and the fermentation time determine the sweetness, alcohol content and acidity of the fermented liquid. If the temperature is too low, the liquid may be too sweet, too much alcohol may be present, or the fermentation time may become too long. If the temperature is too high, there can be an imbalance between yeasts and bacteria, which can prevent the fermentation from going perfectly. If the fermentation time is too short, the conversion of sugars to acids will not continue. If the fermentation time is too long, too much acid will be produced. The present invention greatly increases the chance of obtaining an optimal fermented liquid. This also increases the market value.

According to an embodiment, CO₂ is added externally to the fermented liquid.

In an embodiment, a fermented liquid is bottled in bottles, canned in cans, or stored in large storage vessels such as kegs.

A keg can be part of a tap installation by connecting it to adapted pipes and a tap. This allows fermented liquid to be tapped.

According to an embodiment, one ingredient selected from the group of fruit juices, herbs, extracts, aromas and fruits is optionally added to the fermented liquid. This greatly increases the possible variety of fermented liquid produced. This makes it possible to respond to the different needs and requirements of a consumer, where a basic recipe is maintained, but where the finish of the fermented liquid can be variably chosen. A fermented tea without added ingredients, for example, is also possible, and is called "natural tea".

According to an embodiment, the type of fermented liquid produced is kombucha or jun. Kombucha, also called "Manchurian tea", is a fermented tea made from black or green tea leaves, often touted for its (alleged) health benefits. Also called "Xun", Jun is a fermented tea (similar to kombucha) made from green tea leaves and honey.

It will be apparent to those skilled in the art that the present invention also relates to a method for preventing the formation of a cellulose-based biofilm (SCOBY) during the fermentation of a liquid, for example a fermenting tea. Prevention is achieved by a combination of aeration/addition of oxygen to the fermenting liquid and circulation in the fermentation tank. The embodiments as described above in the context of the production method also apply to this method and are considered to be fully incorporated herein.

In a second aspect, the present invention relates to a device for the industrial production of fermented liquid. The device comprises a closed fermentation tank which is internally provided with a jet head. The fermentation tank is provided with a first conduit which is suitable for transporting at least a portion of the fermenting drink from the fermentation tank to an aerator or oxygenator located outside the fermentation tank.

The first conduit opens into the aerator or oxygenator and a second conduit exits from the aerator or oxygenator and opens into an inlet of the jet head located inside the fermentation tank, wherein the first and second conduits form an external loop located outside the fermentation tank and wherein the jet head is provided with perforations.

This device allows an aerobic fermentation process to be carried out within a closed tank.

According to an embodiment, the fermentation tank is a conical or cylindrically conical fermentation tank, also called a "conical fermenter".

Preferably, a cylindrically conical fermentation tank or conical fermenter is made of stainless steel, where use is made, for example, of grade 304 stainless steel (consisting of 18% chromium and 8% nickel) or grade 316 stainless steel (consisting of 16% chromium, 2% molybdenum and 10% nickel). These possible embodiments are highly resistant to corrosion. Furthermore, the fermentation tank preferably comprises a volume between 100 hL and 4000 hL. More preferably, the content of the fermentation tank is 200 hL.

According to an embodiment, the device comprises a fermentation tank provided with a double wall, wherein the walls define an internal space provided with a cooling liquid (such as for example glycol). This is advantageous to evenly control the temperature in the fermentation tank, optimizing the fermentation process. After all, the use of a cooling liquid around one or more segments of the fermentation tank ensures better heat conduction and a more homogeneous temperature throughout the entire tank. These adjustments allow better control of the fermentation process.

According to an embodiment, the device comprises a heating element for heating the cooling liquid circulating around the fermentation tank. Preferably, a heating element is positioned around one or more tube networks in direct communication with the internal space of the double wall of the fermentation tank. The required temperature, which is indicated according to a method, can be reached by means of the heating element. This is advantageous because the heating element allows to set an optimum temperature for the fermentation, and to correct it during the fermentation if necessary.

The circulation of the liquid in the fermentation tank is done by means of a jet head or mixer. As already mentioned, it has been found that a combination of circulation and aeration with oxygen ensures that the cellulose-based biofilm is not formed in the fermentation tank. Optimal circulation conditions are obtained when a rotary jet head is used. In a preferred form, the jet head will comprise a hollow housing provided with perforations. The jet head is connected to the second pipe, which is the connection between the oxygenator and the fermentation tank, by means of the jet head. This conduit ensures the transfer of the aerated liquid to the fermentation tank and is crucial for moving the fermenting liquid. The jet head receives the liquid transported through the second conduit and distributes it throughout the fermentation tank by means of the perforations. Indeed, a constriction in the inner wall of the jet head causes an acceleration of the liquid flowing through the jet head, which results in a change in the liquid pressure inside the jet head. This effect is also known as the Venturi effect. The velocity built up in the jet head is then transferred from the jet head to the fermentation tank by means of the perforations. This effect causes circulation in the fermentation tank.

The conduits in the fermentation process are preferably made of material suitable for the food industry. These pipes can withstand a pressure of at least 16 bar and are easy to clean. By means of the positions of the valves, the liquid can be passed through the loop between the fermentation tank and oxygenator or sent from the fermentation tank further to the centrifuge, the filter unit and downstream to, for example, a storage unit.

According to a preferred embodiment, the device further comprises one or more separation means, which allow to further purify the obtained fermented liquid from undesirable elements or contaminants. In a preferred form, at least a centrifuge and a filter unit will be present as separation means.

To this end, a centrifuge will be provided, in communication with the fermentation tank, for centrifuging a fermented liquid. The connection between the fermentation tank and downstream units such as the centrifuge is made possible by the conduits and the position of the valves present in the conduits. Preferably, the flow of the fermented liquid will be determined by the position of one or more valves present in the conduits, wherein during the fermentation process a valve allows the liquid to be oxygenated and returned to the fermentation vessel, and wherein upon completion of the fermentation, the position of the one or more valves will be adjusted, thus leading the liquid to a centrifuge.

A centrifugation step allows particles of a certain density or weight to be removed from the fermented liquid. Possible particles include residues of the microorganisms used in the fermentation process.

In a further preferred form, the device comprises a filter unit for filtering the fermented liquid. Preferably, the filter unit is located downstream of the centrifuge. By further filtration through one or more filters, the liquid will, as it were, receive a disinfecting treatment and will be stripped of any microbiological contaminants that can affect the final quality and shelf life of the liquid.

In a preferred form, the filter unit will contain at least three filters. Preferably, a pore size of the filters used is between 0.2 and 20 microns. Filters are preferably made of cellulose acetate, cellulose nitrate, polyamide, polycarbonate, polypropylene or polytetrafluoroethylene. The filter unit comprises at least three filters with different pore sizes, wherein a first filter has a larger pore size than a second filter, and a second filter has a larger pore size than a third filter. Preferably, a 5-10-micron filter is used to obstruct large particles (such as aggregates of biofilm), a 1-5-micron filter (FPEP K402 Europor coarse filter) (FPEP K240 Europor fine filter) is used as a second filter, and a 0.4-0.5-micron filter (FPEP K240 Europor fine filter) is used as the third filter to remove all yeasts and larger bacteria. More preferably, a 0.20-0.28-micron filter (FPEP K470 Europor ultra fine filter) is used to remove all bacteria and achieve better sterility. This is advantageous for removing bacteria and yeasts from the fermented liquid after production. This avoids unwanted fermentation after bottling, whereby an undesired build-up of alcohol and/or CO2 is limited.

The fermented liquid can be bottled by means of a bottling unit. A bottling unit preferably consists of a washing station where empty bottles are cleaned, a filling station where the fermented liquid is poured into bottles, and a closing unit where the bottles with fermented liquid are closed by means of a cap or crown cap. The bottles with fermented liquid can then be provided with a custom label or custom printing. In addition, the fermented liquid can also be stored in vessels, which can be used for tapping the liquid.

In what follows, the invention is described by way of non-limiting examples or figures illustrating the invention, and which are not intended to and should not be interpreted as limiting the scope of the invention.

### DETAILED DESCRIPTION OF THE DRAWINGS

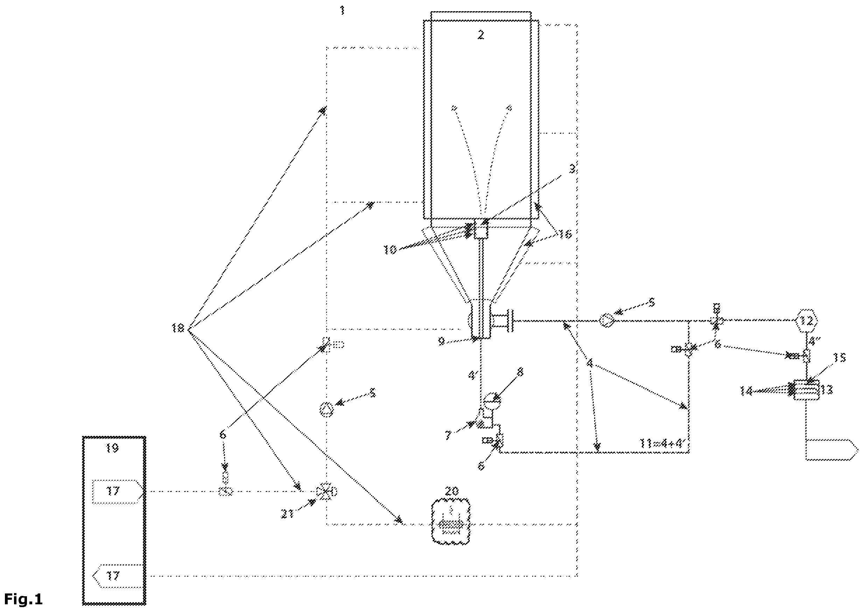

**Figure 1** shows a schematic representation of a device **1** for producing a fermented liquid, according to a possible embodiment of the present invention. The device **1** comprises a fermentation tank **2** which is internally provided with a jet head **3.** The fermentation tank has a volume of 200 hL. It goes without saying that other volumes are also possible.

According to a possible embodiment, the fermentation tank **2** is filled with water (for example tap water) to a certain level. For example, the volume contained in the fermentation tank **2** is maintained at a controlled level of about 200 hL. Then, sugar is added to the water (examples of sugar include glucose, sucrose, or lactose) and the water is brought to a boil. To make a tea infusion, tea leaves are added to the sugar water according to a certain ratio and infused in the sugar water for a certain amount of time, where the time of the tea infusion determines the intensity of the tea. For example, tea leaves may consist of green or black tea, and possibly added loosely to the water. Alternatively, tea is contained in a pouch or bag, wherein the pouch or bag is permeable to water and is added to the water. In an alternative embodiment, a sugar infusion mixture is prepared outside the fermentation tank and transferred to the fermentation tank for fermentation. After the tea incubation, the tea leaves are removed again and the infusion is, in one embodiment, cooled to a temperature between 18°C and 28°C. The symbiotic bacteria (such as for example Gluconobacter or Lactobacillus species) and yeasts (such as for example Saccharomyces) are added to the infusion according to a certain ratio, and the fermentation takes place for 8 to 14 days at a temperature between 18°C and 28°C. The fermenting liquid is circulated in the fermentation tank **2** by means of the jet head **3.**

A first conduit **4** emanating from the fermentation tank allows to divert the contents or part of the contents of the fermentation tank from the fermentation tank. In figure 1, the conduit is provided at the bottom of the fermentation tank, but it goes without saying that other locations on the fermentation tank are also possible, such as at the top or in the middle of the fermentation tank. The direction of liquid flow can be regulated by means of a pump **5** for circulation and valves **6** provided for this purpose. Conduit **4** can send the liquid from the fermentation tank to a more distant processing unit of the device (e.g., a filling unit) or to an oxygenator **7.** This oxygenator **7** allows to supply the fermenting liquid from the fermentation tank with oxygen. To this end, the device **1** is also provided with an oxygen bottle **8** for supplying oxygen to the oxygenator. In another embodiment, not shown, the device will comprise an aerator instead of an oxygenator, and a supply of (compressed) air via a sterile filter.

After the fermenting tea has been mixed with oxygen (or air), the liquid will be returned to the fermentation tank **2** through a second conduit **4'** emanating from the oxygenator **7,** where the conduit **4'** will discharge into an inlet **9** of the jet head **3.** The liquid flow can be regulated again by means of valves **6** provided for that purpose, allowing or preventing a connection. In the fermentation tank **2,** the jet head **3** is provided with perforations **10** with which speed is developed, according to the Venturi effect, with which tea is drawn from the perforations **10** into the jet head **3.** This stimulates circulation.

The first **4** and second **4'** conduit form an external loop **11** which leads back to the jet head inlet **9,** and which finally opens into the jet head **3.** The device **1** thus provides the possibility of circulating fermenting tea from the bottom of the fermentation tank **2,** through the conduits and through the jet head **3.** When tea is flowing through, the jet head **3** can generate at least two liquid flows through the perforations **10** (e.g., two, three or four perforations), whereby circulation is obtained, and the circulation flow rate greatly increases.

In the embodiment shown, the first conduit **4** connects to the oxygenator **7.** In the oxygenator **7,** oxygen from the oxygen bottle **8** is mixed into the fermenting tea. This generates an aeration of the fermenting tea. Subsequently, the aerated tea is returned to the fermentation tank **2** by means of the second conduit **4',** the jet head inlet **9** and the jet head **3,** where the fermentation takes place.

After the fermentation process has been finalized, the liquid can be emptied from the fermentation tank **2** and sent from the fermentation tank via conduit **4** to a unit of the device located further away (for example a filling unit). The first conduit **4** provides the possibility, by means of the pump **5** and the valves **6,** to connect the fermentation tank **2** to a centrifuge **12.** The centrifuge **12** constitutes a first separation step, wherein the fermenting tea is centrifuged. The centrifuge **12** performs a separation, wherein particles or components of a particular density or weight are removed from the fermented tea. The centrifuge **12** is in communication with a downstream positioned filter unit **13** by means of valves **6** in a third conduit **4".** A filter unit **13** forms a second separation step and comprises at least three different filters **14,** wherein the pore size **15** of a filter **14** is between 5 and 0.2 microns. The filter unit **13** is connected, through the valves **6** in the third conduit **4",** downstream to a more distant unit such as a filling station or a storage vessel for the fermented tea (not shown).

In the embodiment shown in figure 1, the fermentation tank **2** comprises a double wall **16,** the walls defining an internal space provided with a cooling liquid **17** (e.g., glycol). A pipe network **18** connects the double wall **16** to the cooling liquid tank **19,** wherein a heating element **20** is present in the pipe network **18** used for heating the cooling liquid. A pump **5** provides the propulsion and valves **6** are present with which a flow of cooling liquid **17** is permitted or prevented. A control valve **21** for determining the cooling temperature is present in the pipe network **18.**

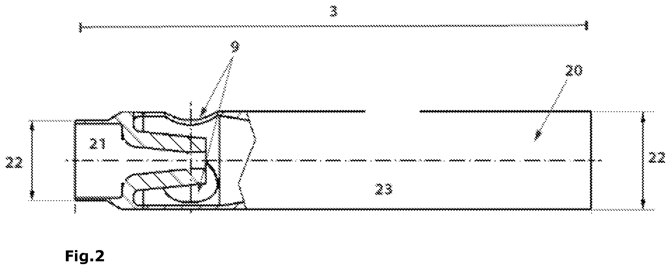

**Figure 2** shows a schematic representation of a jet head **3** which can be used for circulating a fermenting liquid in a closed fermentation tank, according to a possible embodiment of the present invention. The jet head **3** comprises a hollow housing **20** provided with perforations **9.** A first segment **21** of the jet head **3** has a diameter **22** which is 10% to 20% smaller than the diameter **22** of a second segment **23.** The first segment **21** allows a connection to a second conduit **4'** (not visible in figure 3) or is provided with means allowing a conduit 4' to open into the jet head. The perforations **9** are situated in the second segment **23,** in the vicinity of the first segment. Preferably, two to four perforations **9** are present in the jet head **3.** The jet head **3** causes circulation in the fermentation tank **2** by circulating liquid through the perforations **9** in the jet head **3.** At the level of the connection between the first **21** and second **23** segment, the inner wall of the second segment is folded inwards and narrowed. The difference in diameter **22** causes an acceleration of the liquid flowing through the jet head **3,** resulting in a drop in the liquid pressure in the jet head **3.** This effect is also known as the Venturi effect. The pressure built up in the jet head **3** is transferred from the jet head **3** to the fermentation tank **2** by means of the perforations **9,** with the result that circulation in the fermentation tank **2** occurs.

## Claims

1. A method for the industrial production of kombucha based on a fermented liquid, wherein the liquid is a sacchariferous liquid and is fermented by means of microorganisms in a closed fermentation tank, **characterized in that** the fermenting liquid is circulated in the fermentation tank and wherein at least a portion of the fermenting liquid is mixed with oxygen or oxygenated air outside the fermentation tank, and then reintroduced into the fermentation tank.

2. The method according to claim 1, **characterized in that** circulation in the fermentation tank is by means of a mixer or a jet head.

3. The method according to any of the preceding claims, **characterized in that** the mixing of the liquid with oxygen or air is done outside the fermentation tank, wherein a portion of the fermenting liquid is conveyed by means of conduits provided for that purpose that are in connection with the fermentation tank to an aerator or oxygenator, where the fermenting liquid is mixed with oxygen or air, and subsequently conveyed back to the fermentation tank by means of a conduit.

4. The method according to claim 3, **characterized in that** the conduit introducing the aerated or oxygenated liquid back into the fermentation tank discharges into an inlet of the mixer or jet head and the aerated or oxygenated liquid is circulated by means of the mixer or jet head in the fermentation tank.

5. The method according to any of the preceding claims, **characterized in that** the oxygen content in the aerated or oxygenated liquid is between 50 and 8000 ppb.

6. The method according to any of the preceding claims, **characterized in that** the fermented liquid is separated after fermentation, wherein particles or microorganisms of a certain size or density are removed from the fermented liquid.

7. The method according to claim 6, **characterized in that** the fermented liquid undergoes at least two separation steps, wherein a first separation is done by centrifugation and a second separation is done by a filter unit.

8. The method according to claim 7, **characterized in that** a second separation step takes place by means of at least three filters with a pore size between 0.2 to 20 microns, wherein a first filter comprises a pore size greater than a second filter, wherein the second filter comprises a pore size greater than a third filter.

9. The method according to any of the preceding claims, **characterized in that** the liquid is wort or an infusion of green, Oolong or black tea to which sugar or a sugar solution has been added as well as a starter culture of microorganisms.

10. The method according to any of the preceding claims, **characterized in that** a fermentation proceeds by means of an aerobic process.

11. The method according to any of the preceding claims, **characterized in that** the fermentation takes place at a temperature between 18 and 28°C, wherein the fermentation time is preferably 8 to 14 days.

12. The method according to any of the preceding claims, **characterized in that** a starter culture is a mixture of aerobic bacteria and yeasts.

13. A device for the industrial production of kombucha, the device comprising a closed fermentation tank internally provided with a jet head **characterized in that** the fermentation tank is provided with a first conduit suitable for transporting a portion of the fermenting drink from the fermentation tank to an aerator or oxygenator located outside the fermentation tank, wherein the first conduit opens into the aerator or oxygenator and a second conduit exits from the aerator or oxygenator and opens into an inlet of the jet head located inside the fermentation tank, wherein the first and second conduits form an external loop located outside the fermentation tank and wherein the jet head is provided with perforations.

14. The device according to claim 13, **characterized in that** the device comprises a centrifuge, in connection with the fermentation tank, for centrifuging the fermented liquid.

15. The device according to any of the preceding claims, **characterized in that** the device comprises a filter unit for filtering the fermented liquid, wherein the filter unit is located downstream of the centrifuge.

## Patentansprüche

1. Verfahren für die industrielle Produktion von Kombucha, basierend auf einer fermentierten Flüssigkeit, wobei die Flüssigkeit eine zuckerhaltige Flüssigkeit ist und mittels Mikroorganismen in einem geschlossenen Fermentierungstank fermentiert wird, **dadurch gekennzeichnet, dass** die Fermentierungsflüssigkeit in dem Fermentierungstank umgewälzt wird und wobei mindesten ein Teil der Fermentierungsflüssigkeit außerhalb des Fermentierungstanks mit Sauerstoff oder sauerstoffreicher Luft gemischt und dann zurück in den Fermentierungstank geführt wird.

2. Verfahren nach Anspruch 1, **dadurch gekennzeichnet, dass** das Umwälzen in dem Fermentierungstank mittels eines Mischers oder eines Strahlkopfes erfolgt.

3. Verfahren nach einem der vorhergehenden Ansprüche, **dadurch gekennzeichnet, dass** das Mischen der Flüssigkeit mit Sauerstoff oder Luft außerhalb des Fermentierungstanks erfolgt, wobei ein Teil der Fermentierungsflüssigkeit mittels für diesen Zweck bereitgestellter Leitungen, die in Verbindung mit dem Fermentierungstank zu stehen, zu einem Aerator oder Oxygenator befördert wird, wo die Fermentierungsflüssigkeit mit Sauerstoff oder Luft gemischt und danach mittels einer Leitung zurück in den Fermentierungstank befördert wird.

4. Verfahren nach Anspruch 3, **dadurch gekennzeichnet, dass** die Leitung, welche die mit Luft oder Sauerstoff angereicherte Flüssigkeit zurück in den Fermentierungstank führt, in einen Einlass des Mischers oder Strahlkopfes ablässt und dass die mit Luft oder Sauerstoff angereicherte Flüssigkeit mittels des Mischers oder Strahlkopfes in dem Fermentierungstank umgewälzt wird.

5. Verfahren nach einem der vorhergehenden Ansprüche, **dadurch gekennzeichnet, dass** der Sauerstoffgehalt in der mit Luft oder Sauerstoff angereicherten Flüssigkeit zwischen 50 und 8000 ppb beträgt.

6. Verfahren nach einem der vorhergehenden Ansprüche, **dadurch gekennzeichnet, dass** die fermentierte Flüssigkeit nach der Fermentierung abgeschieden wird, wobei Partikel oder Mikroorganismen einer bestimmten Größe oder Dichte aus der fermentierten Flüssigkeit entfernt werden.

7. Verfahren nach Anspruch 6, **dadurch gekennzeichnet, dass** die fermentierte Flüssigkeit mindestens zwei Abscheidungsschritten unterzogen wird, wobei eine erste Abscheidung durch Zentrifugieren und eine zweite Abscheidung durch eine Filtereinheit erfolgt.

8. Verfahren nach Anspruch 7, **dadurch gekennzeichnet, dass** eine zweite Abscheidung mittels mindestens dreier Filter mit einer Porengröße zwischen 0,2 und 20 Mikrometern stattfindet, wobei ein erster Filter eine größere Porengröße als ein zweiter Filter aufweist, wobei der zweite Filter eine größere Porengröße als ein dritter Filter aufweist.

9. Verfahren nach einem der vorhergehenden Ansprüche, **dadurch gekennzeichnet, dass** die Flüssigkeit eine Würze ist oder ein Aufguss von grünem, Oolong- oder schwarzem Tee ist, dem Zucker oder eine Zuckerlösung sowie eine Starterkultur der Mikroorganismen zugesetzt wurde.

10. Verfahren nach einem der vorhergehenden Ansprüche, **dadurch gekennzeichnet, dass** eine Fermentierung mittels eines aeroben Prozesses fortschreitet.

11. Verfahren nach einem der vorhergehenden Ansprüche, **dadurch gekennzeichnet, dass** die Fermentierung bei einer Temperatur zwischen 18 und 28 °C stattfindet, wobei die Fermentierungszeit vorzugsweise 8 bis 14 Tage beträgt.

12. Verfahren nach einem der vorhergehenden Ansprüche, **dadurch gekennzeichnet, dass** eine Starterkultur eine Mischung aus aeroben Bakterien und Hefen ist.

13. Vorrichtung für die industrielle Produktion von Kombucha, wobei die Vorrichtung einen geschlossenen Fermentierungstank umfasst, der in seinem Inneren mit einem Strahlkopf versehen ist, **dadurch gekennzeichnet, dass** der Fermentierungstank mit einer ersten Leitung versehen ist, die zum Transportieren eines Teil des zu fermentierenden Getränks von dem Fermentierungstank zu einem Aerator oder Oxygenator geeignet ist, der sich außerhalb des Fermentierungstanks befindet, wobei die erste Leitung in einen Aerator oder Oxygenator mündet und eine zweite Leitung den Aerator oder Oxygenator verlässt und in einen Einlass des Strahlkopfes mündet, welcher sich im Inneren des Fermentierungstanks befindet, wobei die erste und die zweite Leitung eine externe Schleife bilden, die sich außerhalb des Fermentierungstanks befindet, und wobei der Strahlkopf mit Perforationen versehen ist.

14. Vorrichtung nach Anspruch 13, **dadurch gekennzeichnet, dass** die Vorrichtung eine Zentrifuge in Verbindung mit dem Fermentierungstank zum Zentrifugieren der fermentierten Flüssigkeit umfasst.

15. Vorrichtung nach einem der vorhergehenden Ansprüche, **dadurch gekennzeichnet, dass** die Vorrichtung eine Filtereinheit zum Filtern der fermentierten Flüssigkeit umfasst, wobei sich die Filtereinheit prozessabwärts der Zentrifuge befindet.

## Revendications

1. Procédé de production industrielle de kombucha à base de liquide fermenté, dans lequel le liquide est un liquide saccharifère et est fermenté au moyen de micro-organismes dans une cuve de fermentation fermée, **caractérisé en ce que** le liquide de fermentation circule dans la cuve de fermentation et dans lequel au moins une partie du liquide de fermentation est mélangée avec de l'oxygène ou de l'air oxygéné à l'extérieur de la cuve de fermentation, puis réintroduite dans la cuve de fermentation.

2. Procédé selon la revendication 1, **caractérisé en ce que** la circulation dans la cuve de fermentation se fait au moyen d'un mélangeur ou d'une tête de jet.

3. Procédé selon l'une quelconque des revendications précédentes, **caractérisé en ce que** le mélange du liquide avec de l'oxygène ou de l'air s'effectue à l'extérieur de la cuve de fermentation, dans lequel une partie du liquide de fermentation est acheminée au moyen de conduits prévus à cet effet, qui sont reliés à la cuve de fermentation, vers un aérateur ou un oxygénateur, où le liquide de fermentation est mélangé avec de l'oxygène ou de l'air, puis renvoyé vers la cuve de fermentation au moyen d'un conduit.

4. Procédé selon la revendication 3, **caractérisé en ce que** le conduit réintroduisant le liquide aéré ou oxygéné dans la cuve de fermentation débouche dans une entrée du mélangeur ou de la tête de jet et le liquide aéré ou oxygéné circule au moyen du mélangeur ou de la tête de jet dans la cuve de fermentation.

5. Procédé selon l'une quelconque des revendications précédentes, **caractérisé en ce que** la teneur en oxygène dans le liquide aéré ou oxygéné est comprise entre 50 et 8 000 ppb.

6. Procédé selon l'une quelconque des revendications précédentes, **caractérisé en ce que** le liquide fermenté est séparé après la fermentation, dans lequel des particules ou des micro-organismes d'une certaine taille ou densité sont éliminés du liquide fermenté.

7. Procédé selon la revendication 6, **caractérisé en ce que** le liquide fermenté subit au moins deux étapes de séparation, dans lequel une première séparation est effectuée par centrifugation et une seconde séparation est effectuée par une unité de filtration.

8. Procédé selon la revendication 7, **caractérisé en ce qu'**une seconde étape de séparation a lieu au moyen d'au moins trois filtres ayant une taille de pores comprise entre 0,2 et 20 microns, dans lequel un premier filtre comprend une taille de pores supérieure à celle d'un deuxième filtre, dans lequel le deuxième filtre comprend une taille de pores supérieure à celle d'un troisième filtre.

9. Procédé selon l'une quelconque des revendications précédentes, **caractérisé en ce que** le liquide est du moût ou une infusion de thé vert, Oolong ou noir auquel a été ajouté du sucre ou une solution sucrée ainsi qu'un levain de micro-organismes.

10. Procédé selon l'une quelconque des revendications précédentes, **caractérisé en ce qu'**une fermentation se déroule au moyen d'un processus aérobie.

11. Procédé selon l'une quelconque des revendications précédentes, **caractérisé en ce que** la fermentation a lieu à une température comprise entre 18 et 28 °C, dans lequel la durée de fermentation est de préférence de 8 à 14 jours.

12. Procédé selon l'une quelconque des revendications précédentes, **caractérisé en ce qu'**un levain est un mélange de bactéries aérobies et de levures.

13. Dispositif pour la production industrielle de kombucha, le dispositif comprenant une cuve de fermentation fermée munie intérieurement d'une tête de jet, **caractérisé en ce que** la cuve de fermentation est munie d'un premier conduit approprié pour transporter une partie de la boisson en fermentation depuis la cuve de fermentation vers un aérateur ou un oxygénateur situé à l'extérieur de la cuve de fermentation, dans lequel le premier conduit débouche dans l'aérateur ou l'oxygénateur et un second conduit sort de l'aérateur ou de l'oxygénateur et débouche dans une entrée de la tête de jet située à l'intérieur de la cuve de fermentation, dans lequel les premier et second conduits forment une boucle externe située à l'extérieur de la cuve de fermentation et dans lequel la tête de jet est munie de perforations.

14. Dispositif selon la revendication 13, **caractérisé en ce que** le dispositif comprend une centrifugeuse, en liaison avec la cuve de fermentation, pour centrifuger le liquide fermenté.

15. Dispositif selon l'une quelconque des revendications précédentes, **caractérisé en ce que** le dispositif comprend une unité de filtrage pour filtrer le liquide fermenté, l'unité de filtrage étant située en aval de la centrifugeuse.
